(19) Europäisches Patentamt
European Patent Office
Office européen des brevets

(11) **EP 4 700 008 A1**

(12) **EUROPEAN PATENT APPLICATION**

(43) Date of publication:
**25.02.2026 Bulletin 2026/09**

(21) Application number: **24306368.2**

(22) Date of filing: **18.08.2024**

(51) International Patent Classification (IPC):
**C07C 45/52** [(2006.01)]    **C07C 47/22** [(2006.01)]
**B01J 21/08** [(2006.01)]    **B01J 27/02** [(2006.01)]

(52) Cooperative Patent Classification (CPC):
(C-Sets available)
**C07C 45/52; B01J 21/08; B01J 27/02;
B01J 27/188; B01J 35/30; B01J 35/40;
B01J 35/80;** B01J 35/63      (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Technip Energies France
92741 Nanterre Cedex (FR)**

(72) Inventor: **Rodriguez, Mariam Salazar
92741 Nanterre Cedex (FR)**

(74) Representative: **Edson, Russell Gregory et al
Withers & Rogers LLP
2 London Bridge
London SE1 9RA (GB)**

(54) **PRODUCTION OF ACROLEIN FROM GLYCEROL**

(57)      A method of producing acrolein from glycerol is disclosed. The method may include introducing a heterogenous acid catalyst into a reactor. The heterogenous acid catalyst may be a heteropoly acid catalyst or a sulfonic acid catalyst. The heterogenous acid catalyst may be heated to a target reaction temperature while in the reactor. In some cases, the heterogenous acid catalyst may be preheated in the reactor before being heated to the target reaction temperature. Once the heterogenous acid catalyst reaches the target reaction temperature, glycerol may be introduced into the reactor to contact the heterogenous acid catalyst. Contact of the glycerol with the heterogenous acid catalyst causes a reaction that dehydrates the glycerol in the reactor and produces an acrolein-containing effluent. The effluent may be discharged from the reactor and may be subjected to further downstream processing, including converting the acrolein into acrylic acid.

FIG. 1

**EP 4 700 008 A1**

(52) Cooperative Patent Classification (CPC): (Cont.)

C-Sets
**C07C 45/52, C07C 47/22**

**Description**

**Technical Field**

The present disclosure relates generally to acrolein production and more particularly although not necessarily exclusively, to the production of acrolein in a reactor by dehydrating an oxygenate feedstock in the presence of a heterogenous catalyst.

**Background**

Acrolein is regularly used as an intermediate in the preparation of acrylic acid. One major application for acrylic acid is as a feedstock for commodity esters, such as for example, methyl acrylate, ethyl acrylate, butyl acrylate, and 2-ethylhexyl acrylate. Acrylate esters are used in various industries and for numerous purposes. For example, acrylate esters are commonly used in paints, surface coatings, adhesives, sealants, textiles, plastics, additives, and in the treatment of paper. A significant amount of manufactured crude acrylic acid is alternatively upgraded and purified into glacial acrylic acid for the production of polyacrylic acid or copolymers, which are commonly used as super absorbents that are routinely found in products such as disposable diapers, detergent co-builders, dispersants, flocculants and thickeners. The preferred and dominant method of acrylic acid production is two-stage propylene oxidation, whereby propylene is converted to acrolein and acrolein is converted to acrylic acid. Propylene is typically manufactured using fossil fuels. Therefore, sustainable alternatives to producing acrylic acid using propylene are needed.

**Summary**

Example 1 is a method of producing acrolein from glycerol. The method includes introducing into a reactor, a heteropoly acid catalyst comprising amorphous silica having a heteropoly acid group. The method also includes preheating the heteropoly acid catalyst in the reactor to a temperature within a temperature range of between 150 °C to 250 °C and maintaining the temperature of the heteropoly acid catalyst within the temperature range for a predetermined period of time to produce a preheated heteropoly acid catalyst. The method additionally includes further heating the preheated heteropoly acid catalyst to a target reaction temperature, introducing glycerol into the reactor and dehydrating the glycerol in the reactor by a reaction of the glycerol with the heteropoly acid catalyst that produces an acrolein-containing effluent, and discharging the acrolein-containing effluent from the reactor.

Example 2 is Example No. 1, wherein a pore volume (PV) of the heteropoly acid catalyst satisfies a formula: PV > 0.6-0.3 [loading/surface area of dried heterogenous acid catalyst], where PV is measured in milliliters per gram of catalyst, the loading is an amount of heterogenous acid present in the dried heterogenous acid catalyst and is measured in micromoles per gram, and the surface area of dried heterogenous acid catalyst is measured in square meters per gram of the heterogenous acid catalyst.

Example 3 is Example No. 1, wherein the glycerol is vapor phase glycerol or aqueous glycerol.

Example 4 is Example No. 1, wherein the heteropoly acid catalyst has an acid dissociation constant (pKa) value of 3.7 or less.

Example 5 is Example No. 1, wherein the heteropoly acid catalyst is selected from the group consisting of tungsten-based heteropoly acids including tungstosilicic acid, tungstophosphoric acid, and phosphomolybdic acid, and salt-based heteropoly acids including 12-tungstosilicic acid and 18-tungstiphosphoric acid.

Example 6 is Example No. 1, wherein the amorphous silica contains between zero to 0.05 weight percent of zirconium, between zero to 0.035 weight percent of sodium, between zero to 40 parts per million of iron, and between zero to 60 parts per million of molybdenum.

Example 7 is Example No. 1, wherein the target reaction temperature is between 220 °C to 300 °C.

Example 8 is Example No. 1, wherein the reaction occurs in a vapor phase state.

Example 9 is Example No. 1, wherein a pressure in the reactor at a time of the reaction is at least 0.1 MPa below a dew point pressure, and at least 10 °C above a dew point temperature, of both the glycerol and the acrolein-containing effluent produced by the reaction.

Example 10 is Example No. 1, wherein the glycerol contains water or is combined with water to regulate an endothermic reaction temperature profile inside the reactor.

Example 11 is a method of producing acrolein from glycerol. The method includes introducing into a reactor, a sulfonic acid catalyst comprising amorphous silica having organosulfonic acid groups chemically bonded thereto and having an acid dissociation constant (pKa) value of 3.5 or less, and heating the sulfonic acid catalyst to a target reaction temperature. The method further includes introducing glycerol into the reactor and dehydrating the glycerol in the reactor by a reaction of the glycerol with the sulfonic acid catalyst that produces an acrolein-containing effluent, and discharging the acrolein-containing effluent from the reactor.

Example 12 is Example No. 11, wherein the glycerol is vapor phase glycerol or aqueous glycerol.

Example 13 is Example No. 11, wherein the target reaction temperature is between 220 °C to 300 °C.

Example 14 is Example No. 11, wherein the reaction occurs in a vapor phase state.

Example 15 is Example No. 11, wherein a pressure in the reactor at a time of the reaction is at least 0.1 MPa below a dew point pressure, and at least 10 °C above a dew point temperature, of both the glycerol and the acrolein-containing effluent produced by the reaction.

Example 16 is Example No. 11, wherein the glycerol contains water or is combined with water to regulate an endothermic reaction temperature profile inside the reactor.

Example 17 is a catalyst for producing acrolein from glycerol. The catalyst includes an amorphous silica having organosulfonic acid groups chemically bonded thereto, and has an acid dissociation constant (pKa) value of 3.5 or less.

Example 18 is Example No. 17, wherein the sulfonic acid catalyst is selected from the group consisting of methanesulfonic acid silica, ethanesulfonic acid silica, propanesulfonic acid silica, butanesulfonic acid silica, benzenesulfonic acid silica, ethylbenzenesulfonic acid silica, vinylbenzenesulfonic acid silica, propylbenzenesulfonic acid silica, and butylbenzene sulfonic acid silica.

Example 19 is Example No. 17, wherein the amorphous silica of the sulfonic acid catalyst is functionalized with one or more organosulfonic acid groups having a formula R1SO3H, and the R1 is chemically bonded to the amorphous silica and is selected from the group consisting of: a substituted alkyl group, a substituted alkenyl group, and a substituted alkynyl group, each having between 1 to 8 carbon atoms, and a substituted or unsubstituted aryl group.

Example 20 is Example No. 17, wherein the amorphous silica comprises silica particles in the form of shaped objects in which the silica particles are composited together with or without the aid of a binder.

## Brief Description of the Drawings

FIG. 1 is a schematic diagram illustrating one example configuration of a pilot plant for producing acrolein from glycerol.

FIG. 2 is a graph illustrating the performance of a heteropoly acid catalyst relative to producing acrolein from glycerol according to one example.

FIG. 3 is a table reporting various testing data for the heteropoly acid catalyst associated with FIG. 2 when preheated to different temperatures according to one example.

FIG. 4 is a table reporting additional testing data and comparing the preheated heteropoly acid catalyst examples associated with FIG. 3 to several alternative catalysts according to one example.

FIG. 5 is a graph illustrating the performance of the heteropoly acid catalyst associated with FIG. 2 relative to producing acrolein from glycerol after preheating the heteropoly acid catalyst to a second temperature according to one example.

FIG. 6 is a graph illustrating the performance of the heteropoly acid catalyst associated with FIG. 2 relative to producing acrolein from glycerol after preheating the heteropoly acid catalyst to a third temperature according to one example.

FIG. 7 is a graph illustrating the performance of a first alternative heteropoly acid catalyst relative to producing acrolein from glycerol according to one example.

FIG. 8 is a graph illustrating the performance of a second alternative heteropoly acid catalyst relative to producing acrolein from glycerol according to one example.

FIG. 9 is a graph illustrating the performance of a third alternative heteropoly acid catalyst relative to producing acrolein from glycerol according to one example.

FIG. 10 is a graph illustrating the performance of a sulfonic acid catalyst relative to producing acrolein from glycerol according to one example.

FIG. 11 is a table reporting various testing data associated with multiple sulfonic acid catalyst compositions according to one example.

FIG. 12 is a thermogravimetric analysis graph for the sulfonic acid catalyst associated with FIG. 10 after preheating according to one example.

FIG. 13 is a flowchart describing one example of a method of producing acrolein from glycerol.

## Detailed Description

Certain aspects and examples of the present disclosure relate to a system and method for producing acrolein by dehydrating alcohol, diol, or triol compounds to unsaturated oxygenates such as aldehydes. Particularly, aspects and examples of the present disclosure relate to a system and method for producing acrolein from glycerol. As with alcohol, diol, or triol compounds, glycerol may be obtained from renewable plant-based sources such as, for example, as a byproduct of biodiesel production where it is produced by the transesterification process of vegetable oils or animal fats. Therefore, producing acrolein using glycerol lacks the drawbacks associated with producing acrolein from a non-renewable feedstock such as propylene.

The reaction required to produce acrolein from glycerol involves dehydrating an oxygenate feedstock - particularly glycerol - in a reactor in the presence of a heterogenous catalyst under specific conditions. The reaction can be carried out in a liquid phase or in gas phase.

In some examples, the heterogenous catalyst may be a heterogenous acid catalyst. In one example, the heterogenous acid catalyst may be a heteropoly acid catalyst comprising amorphous silica functionalized with a heteropoly acid group. In another example, the heterogenous acid catalyst may be a sulfonic acid catalyst comprising amorphous silica having organosulfonic acid groups chemically bonded thereto. In either case, the amorphous silica may comprises silica particles, in the form of shaped objects, such as extrudates, in which the silica particles are composited together with or without the aid of a binder. The term "amorphous," as used herein to describe the aforementioned acid catalysts, is used in its commonly accepted sense to mean lacking long range order such as would give rise to one or more intense peaks in an X-ray diffraction pattern.

In some examples where a heteropoly acid catalyst is used, the acrolein production process can include a pretreatment procedure wherein the heteropoly acid catalyst is preheated to a temperature within a certain temperature range before being finally heated to a target reaction temperature. In some examples, the acrolein production process can utilize temperature and pressure ranges that cause the process to occur in a vapor phase state. In some examples, the acrolein production process may be performed at a reaction pressure that is at least some amount below the dew point pressure, and at a reaction temperature that is at least some amount above the dew point temperature, of both the glycerol and an acrolein-containing gaseous effluent produced by the reaction. In some examples, the glycerol may contain water or may be combined or mixed with water to regulate an endothermic reaction temperature profile inside the reactor. In some examples, a glycerol evaporation pretreatment step may be used to eliminate impurities present in a aqueous solution of glycerol. In an example, the reaction may be performed in an adiabatic reactor. In another example, the process may be performed in an isothermal reactor.

Illustrative examples follow and are given to introduce the reader to the general subject matter discussed herein rather than to limit the scope of the disclosed concepts. The following sections describe various additional features and examples with reference to the drawings in which like numerals indicate like elements, and directional descriptions are used to describe the illustrative aspects, but, like the illustrative aspects, should not be used to limit the present disclosure.

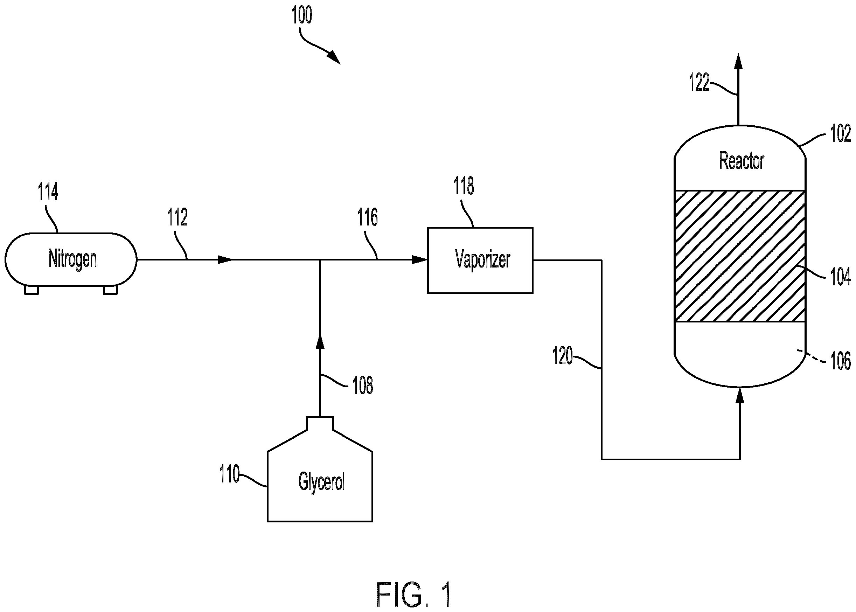

FIG. 1 is a schematic diagram of one example of a pilot plant 100 for producing acrolein by dehydrating a compound such as an alcohol, a diol, or a triol, and particularly glycerol. The pilot plant 100 is configured to carry out a dehydration reaction in a continuous mode in a reactor 102. In at least some examples, the reactor 102 may include a fixed catalyst bed 104 and a reactor-operated down-flow. In one example configuration, the reactor 102 may be an adiabatic reactor. In another example configuration, the reactor 102 may be an isothermal reactor.

In one example of an acrolein production process using the pilot plant 100, a catalyst 106 may be initially directed to or otherwise placed in the reactor 104. In an example, a flow of aqueous glycerol 108 from an aqueous glycerol source 110 (e.g., storage tank) can be directed to a vaporizer 112 to produce vapor phase glycerol 114 that may be subsequently introduced to the reactor 102.

Once the vapor phase glycerol 114 has been introduced to the reactor 102, dehydration of the glycerol can occur in the reactor 102 through contact of the vapor phase glycerol 114 with the catalyst 106. The glycerol can be dehydrated in the reactor according to the following reaction:

$$CH_2OH\text{-}CHOH\text{-}CH_2OH \rightarrow CH_2=CH\text{-}CHO + 2H_2O$$

The reaction is carried out in a vapor phase in this example, but may instead be carried out in a liquid phase in other examples. In either case, the reaction can produce a high yield of acrolein. In the example of FIG. 1, the result of the glycerol dehydration process performed in the reactor 102 is an acrolein-containing effluent 116 of a gaseous form. When the reaction is carried out in the liquid phase, the result of the glycerol dehydration process can be an acrolein-containing effluent 116 of a liquid form. The acrolein-containing effluent 116 can be directed from the reactor 102 to other downstream processes, such as for example, cooling, quenching, distillation, and/or various separation processes via which the acrolein can be converted to other compounds such as but not limited to acrylic acid.

In one example, the catalyst 106 may be amorphous silica functionalized to have a heteropoly acid group (hereinafter also referred to as a "heteropoly acid catalyst"). A "heteropoly acid" according to the disclosure is an acid comprising a combination of hydrogen and oxygen and including an addenda atom supplied by a metal (e.g., tungsten) and a hetero atom (e.g., phosphorous). The heteropoly acid can be a salt, such as for example, a cation salt or a metal salt. In some examples, the heteropoly acid can be a mixture of different heteropoly acids or salts. In some examples, the addenda atom may be, instead of tungsten, another polyvalent metal such as molybdenum, niobium, tantalum, tungsten, or vanadium. In some examples, the hetero atom may be, instead of phosphorous, silicon or any of a number of different Group I-VIII elements.

When the catalyst 106 is a heteropoly acid catalyst, the heteropoly acid catalyst preferably has an acid dissociation constant (pKa) value of 3.7 or less. The amorphous silica of the heteropoly acid catalyst may comprise silica particles in the form of shaped objects, such as extrudates, in which the silica particles are composited together with or without the aid of a binder. The amorphous silica of the heteropoly acid catalyst is preferably also substantially free of contaminants. For example, the amorphous silica may contain between zero to 0.05 weight percent of zirconium, or more preferably, no measurable amount of zirconium. Similarly, the amorphous silica may contain between zero to 0.035 weight percent of sodium, and more preferably, no measurable amount of sodium. The amorphous silica may preferably contain between zero to 40 parts per million of iron and, and between zero to 60 parts per million of molybdenum. Thus, the heteropoly acid may be supported in substantially pure silica.

When the catalyst 106 is a heteropoly acid catalyst, the pore volume (PV) of the heteropoly acid catalyst preferably satisfies the formula:

$$PV > 0.6\text{-}0.3 \text{ [loading/surface area of dried heteropoly acid catalyst]} \qquad (1)$$

where PV is measured in milliliters per gram of catalyst; the loading is the amount of heteropoly acid present in the dried heteropoly acid catalyst, measured in micromoles per gram; and the surface area of dried heteropoly acid catalyst is measured in square meters per gram of catalyst.

In some examples, the heteropoly acid may particularly comprise Group 6 elements. For example, the heteropoly acid catalyst may be a tungsten-based heteropoly acid such as tungstosilicic acid, tungstophosphoric acid, or phosphomolybdic acid. Alternatively, the heteropoly acid catalyst may be a sodium-based heteropoly acid such as, for example, 12-tungstosilicic acid or 18-tung

In some examples, a selected heteropoly acid can be dissolved in a solvent as part of a process for producing the heteropoly acid catalyst. The solvent may include, for example, alcohols, aldehydes, ethanol, water such as distilled water, and a number of other possible solvents in which a heteropoly acid can be dissolved. The amorphous silica can then be functionalized by applying the dissolved heteropoly acid-solvent mixture thereto. For example, the heteropoly acid can be added to the amorphous silica by immersing the amorphous silica in the dissolved heteropoly acid-solvent mixture or by otherwise impregnating the amorphous silica with the dissolved heteropoly acid-solvent mixture. The heteropoly acid catalyst may thereafter be finally produced by, for example, drying the heteropoly acid-impregnated amorphous silica. The drying step may occur at an elevated temperature over a period of time, and may be followed by a cooling period prior to using the heteropoly acid catalyst to produce acrolein from glycerol.

In another example, the catalyst 106 may be amorphous silica having organosulfonic acid groups chemically bonded thereto (hereinafter also referred to as a "sulfonic acid catalyst"). When the catalyst 106 is a sulfonic acid catalyst, the sulfonic acid catalyst preferably has an acid dissociation constant (pKa) value of 3.5 or less. The amorphous silica of the sulfonic acid catalyst may also comprise silica particles in the form of shaped objects, such as extrudates, in which the silica particles are composited together with or without the aid of a binder.

When the catalyst 106 is a sulfonic acid catalyst, the organosulfonic acid groups chemically bonded to the amorphous silica may have the formula $R^1SO_3H$, where $R^1$ is chemically bonded to the amorphous silica and can comprise a substituted alkyl group, alkenyl group, or alkynyl group, preferably having up to 8 carbon atoms, or a substituted or unsubstituted aryl group. In some examples, $R^1$ may be an alkyl group, such as an alkyl group having 1 to 4 carbon atoms. Such functionalized silicas may be referred to herein as arylsulfonic acid silicas.

Examples of suitable organosulfonic acid functionalized silica compounds having a pKa value of 3.5 or less may include, for example, methanesulfonic acid silica, ethanesulfonic acid silica, propanesulfonic acid silica, butanesulfonic acid silica, benzenesulfonic acid silica, ethylbenzenesulfonic acid silica, vinylbenzenesulfonic acid silica, propylbenzenesulfonic acid silica, and butylbenzene sulfonic acid silica. Usable organosulfonic acid functionalized silica compounds having a pKa value of 3.5 or less may be obtained from commercial sources, or suitable organosulfonic acid functionalized silica compounds can be synthesized. In one example, an existing amorphous silica (support) can be post-functionalized by reaction of silanol groups on the amorphous silica with an alkoxysilane containing a thiol group such as, for example, 3-mercaptopropyltrimethoxysilane (MPTMS). In another example, a functionalized silica compound can be synthesized by co-condensation of an alkoxysilane containing a thiol group, such as MPTMS, with a silicon source, such as for example, siloxane precursors (e.g., tetraethyl orthosilicate, TEOS, or tetramethyl orthosilicate, TMOS). Oxidation of the thiol groups to sulfonic acids can then be accomplished using oxidants, such as for example, hydrogen peroxide ($H_2O_2$).

In some examples where the catalyst 106 is a heteropoly acid catalyst, a pretreatment procedure may be employed and may include preheating the heteropoly acid catalyst before reacting the glycerol 114 therewith in the reactor 102. In some examples, the heteropoly acid catalyst may be preheated in the reactor 102 to a temperature of between 150 °C to 250 °C. For example, the heteropoly acid catalyst may be preheated in the reactor 102 to a temperature of between 220 °C to 230 °C. The elevated temperature of the heteropoly acid catalyst may be maintained for a sufficient time (e.g., one hour) to remove bound water from the heteropoly acid component of the heteropoly acid catalyst. The elevated temperature may be maintained under an anhydrous atmosphere. The preheating process results in a preheated heteropoly acid catalyst. Once the preheating process is complete (e.g., the bound water has been removed), the temperature of the heteropoly acid catalyst can be raised to a target temperature for the reaction of the glycerol 114 therewith.

In some examples, the target temperature for the reaction of the glycerol 114 with the catalyst 106 in the reactor 102 is controlled such that the dehydration reaction of the glycerol is performed in a vapor phase state. In one example, the target temperature for the reaction of the glycerol 114 with the catalyst 106 in the reactor 102 is controlled such that the dehydration reaction of the glycerol is performed within a temperature range of between 220 °C to 300 °C. In some examples, the reaction is controlled such that the pressure in the reactor 102 at the time the glycerol 114 is contacted with the catalyst 106 is at least 0.1 MPa below a dew point pressure, and at least 10 °C above a dew point temperature, of both the glycerol 114 and the acrolein-containing effluent 116 produced by the reaction.

In an example, the glycerol 114 may contain water to regulate an endothermic reaction temperature profile inside the reactor 102. In another example, the glycerol 114 may be combined or mixed with water to regulate the endothermic reaction temperature profile inside the reactor 102. In some examples, the aqueous glycerol 108 may be pretreated to remove impurities prior to vaporization in the vaporizer 112. Impurities that may be removed by such a pretreatment step may include, for example, one or more of sodium chloride, sodium sulfate, non-glycerin organic matter, and methanol. In one example, the reactor 102 may be an adiabatic reactor where there is substantially no transfer of heat between the reacting materials in the reactor and the surrounding environment. In another example, the reactor 102 may be an isothermal reactor where there is a transfer of heat between the reacting materials in the reactor and the surrounding environment, which can allow the reaction temperature to remain substantially constant.

Example 1 - Heteropoly Acid Catalyst Performance

The pilot plant 100 was used to test the process for producing acrolein from glycerol as generally described above. In order to monitor the process and to gather data for analysis, three acrolein detectors were located at the pilot plant 100. An experimental glycerol dehydration reaction was performed in continuous mode within the reactor 102 by vaporizing 20 weight percent of the aqueous glycerol 108 in the vaporizer 112 to produce the vapor phase glycerol 114 for introduction to the reactor 102. A chemical analysis of the effluent 116 was performed on-line using a flame ionization detection gas chromatography (FID-GC) detector provided with a Q-Plot column. Quantitative analysis was done by obtaining calibration curves for each main detected component. The calibration curves allowed for establishment of the ratio between the gas chromatograph response (area) vs. known concentrations of prepared solutions. Off-line analysis using a gas chromatograph coupled with a mass spectrometer (MS) was also performed to validate the on-line analysis, but only results from the on-line analysis are provided herein.

The experimental glycerol dehydration reaction according to this example was performed using 1 gram of a heteropoly acid catalyst. The catalyst was prepared using silicotungstic acid and a high-purity porous silica support such that the pore volume (PV) of the supported silicotungstic acid catalyst satisfied Equation 1. The acid dissociation constant (pKa) value of the heteropoly acid catalyst was 3.6, and the particle sizes of the silica were between 500 $\mu$m to 800 $\mu$m. The heteropoly acid catalyst was diluted 1:1 with silicon carbide (SiC) to facilitate effective flow distribution and heat transfer.

Once in the reactor 102, the heteropoly acid catalyst was preheated at a temperature of 220 °C for one hour and then further heated to the target temperature for the dehydration reaction prior to the start thereof. Once the target reaction temperature was achieved, the vapor phase glycerol 114 was introduced to the reactor 102 at the selected reaction conditions, which were controlled to ensure that contact of the glycerol with the heteropoly acid catalyst occurred in a vapor phase state. The weight hour space velocity (WHSV) was set at 3 h-1, the glycerol partial pressure was 3E-02 atmospheres, and the flow of glycerol was maintained at 3 gallons per hour. The reaction was performed at atmospheric pressure.

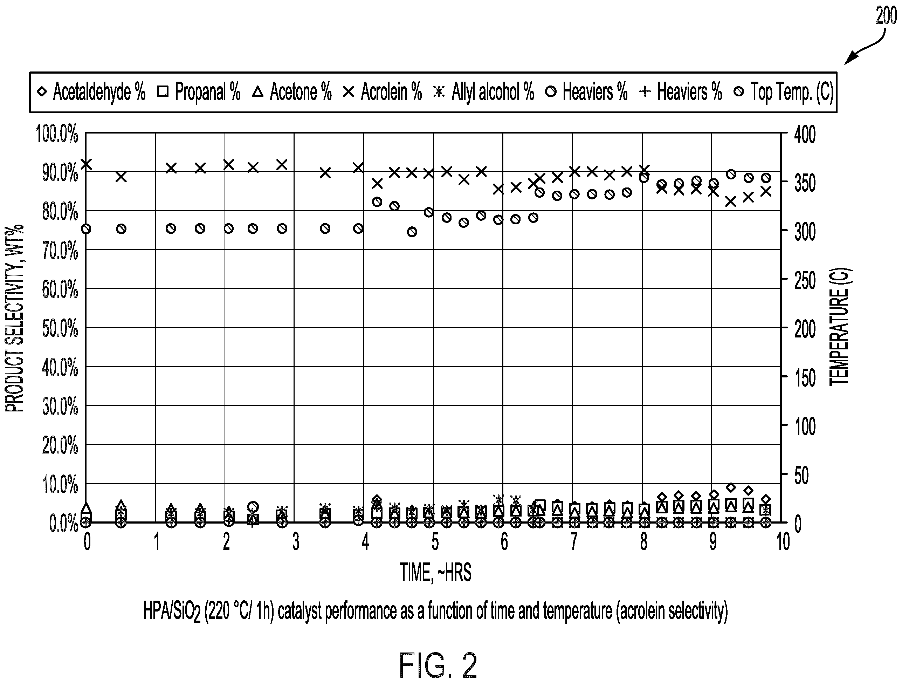

FIG. 2 is a graph 200 illustrating the performance of the silicon carbide-diluted heteropoly acid catalyst relative to the production of acrolein from the glycerol 114. As shown, once the reactor 102 reached steady state, an acrolein selectivity of over 90% was achieved at a reaction temperature of 300 °C. It may also be observed that the acrolein selectivity decreased slightly when the reaction temperature was increased to 350 °C. Analysis by gas chromatograph detected no glycerol in the reactor effluent 116. The primary by-products in the effluent 116 were determined to be acetaldehyde and acetone.

Example 2 - Effect of Thermal Treatment on Heteropoly Acid Catalyst Performance

This heteropoly acid catalyst test followed a similar procedure as described in Example 1 relative to testing of the heteropoly acid catalyst performance in producing acrolein. In this example, however, the heteropoly acid catalyst used was dried by preheating to temperatures greater than the 220 °C preheat temperature of Example 1 and then introduced into the reactor 102 as part of separate test reactions to determine if the heat treatment temperature affects the acrolein production process. The selected heteropoly acid catalyst preheating temperatures were 220 °C, 400 °C, and 550 °C, and the preheating in each case lasted for one hour. The preheating temperatures facilitated the removal of bound water from the heteropoly acid component of the supported heteropoly acid catalyst, under an anhydrous atmosphere. In each separate test reaction, the heteropoly acid catalyst was further heated in the reactor 102 to the target reaction temperature after the preheating operation and a glycerol dehydration reaction was then performed in continuous mode within the reactor 102 by vaporizing 20 weight percent of the aqueous glycerol 108 in the vaporizer 118 to produce the vapor phase glycerol 114, and then introducing the vapor phase glycerol 114 into the reactor 102. The space velocity WHSV was set at 3 h-1, the glycerol partial pressure was 3E-02 atm and the reaction was performed at atmospheric pressure.

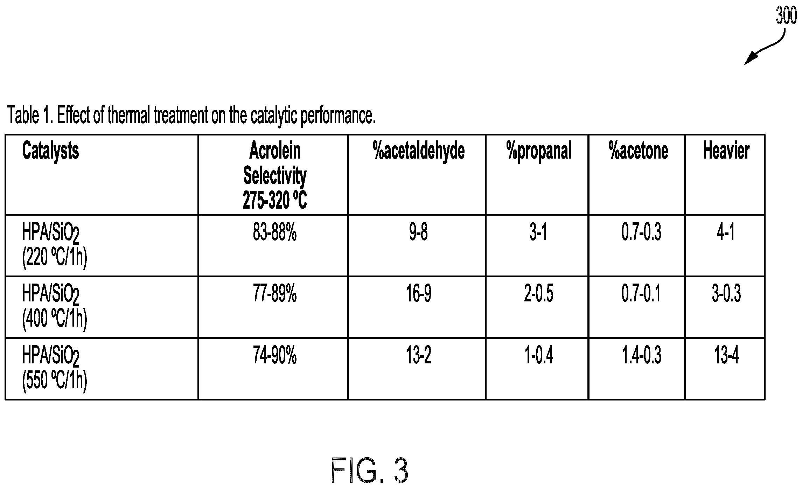

FIG. 3 includes a table 300 of testing data and is labeled as Table 1. The testing data reveals the performance of each of the three preheated heteropoly acid catalysts relative to producing acrolein from the vapor phase glycerol 114 at a reaction temperature range of between 275 °C to 320 °C. It may be observed in Table 1 of FIG. 3 that the average range of acrolein selectivity decreases with an increase in the heteropoly acid catalyst preheating temperature. Additionally, increases in the heteropoly acid catalyst preheating temperature correspond with increases in effluent byproducts. The primary byproducts in this example were found to be acetaldehyde, propanal, acetone and heavier byproducts, the latter of which was significantly affected by the heteropoly acid catalyst preheating temperature. It is also noted that when the heteropoly acid catalyst preheated to a temperature of 550 °C was used as the catalyst for the dehydration reaction, full conversion of glycerol was not achieved, as indicated by the presence of a corresponding glycerol peak in the chromatogram generated by the gas chromatograph. The loss of activity and selectivity can be explained by the decomposition and agglomeration of the active species which are induced at high temperatures.

Example 3 - Effect of Acid Strength on Heteropoly Acid Catalyst Performance

According to Example 3, the heteropoly acid catalysts pre-treated at the three different temperatures as described in Example 2 were tested for acrolein production performance against three other catalysts according to the dehydration reaction procedure described in Example 1. In this example, the three other catalysts were the zeolite Mordenite Si/Al 40 (MOR 40) (Catalyst A), the zeolite Chabazite (SSZ-13) (Catalyst B), and a material referred to herein as $WO_3$/AlOOH (Catalyst C). The $WO_3$/AlOOH material was prepared in-house by wet impregnation of ammonium paratungstate with a monolayer coverage between 10-15% of boehmite (AIOOH), which was used as a catalyst support. Once the impregnation was completed, the resulting material was dried and calcined at 400 °C for 4 hours. Acid dissociation constant (pKa) values for each catalyst were measured by titration using a dispersion of 0.15 grams of the catalyst in 40 grams of a sodium chloride/water solution (2.5 weight percent). The resulting slurry was left for a minimum of 4 hours under agitation. Titration was subsequently performed using a solution of sodium hydroxide (0.1-0.01 N). The pKa value for each catalyst was determined at the half titration point in the titration curve. At this point, the concentrations of base and acid are equal, and therefore the pKa value is equivalent to the pH value.

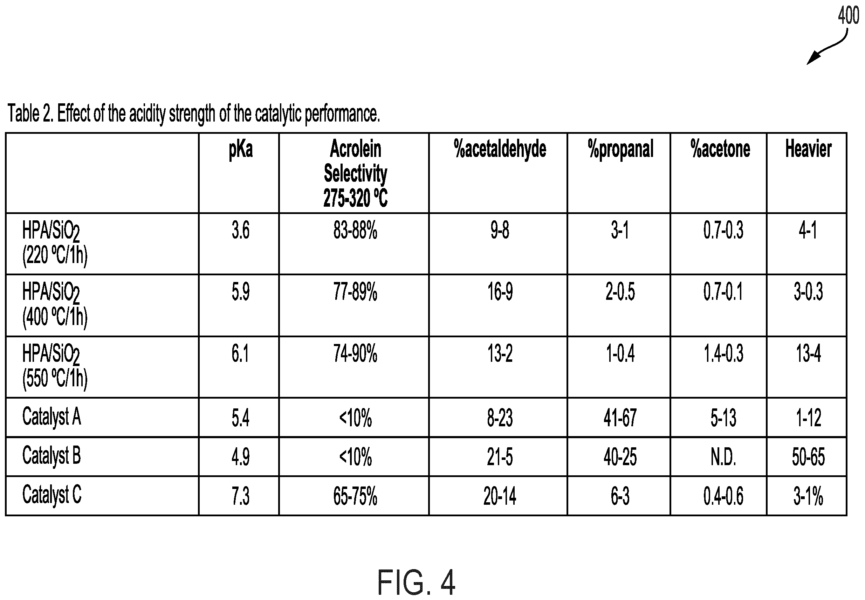

FIG. 4 includes a table 400 of test data and is labeled as Table 2. The average pKa value for each catalyst is reported in Table 2 in units of milliequivalents per gram (meq/g). The catalytic performance exhibited by each catalyst when used in a glycerol dehydration process at temperatures ranging from 275 °C to 320 °C is also reported in Table 2 . As indicated, acrolein selectivity generally correlates strongly with the strength of the catalyst. For example, it may be observed that the acrolein selectivity is high (between 83% to 88%) when the pKa value of the heteropoly acid catalyst is low (e.g., 3.6). In contrast, Catalyst A and Catalyst B, which had pKa values of 5.4 and 4.9 respectively, each had an acrolein selectively of less than 10%. This is not particularly surprising given that Catalyst A and Catalyst B comprise structured zeolites, which are prone to form propanal. While Catalyst C had the highest pKa value of the tested materials, at 7.3, Catalyst C nonetheless exhibited better acrolein selectivity that Catalyst A and Catalyst B, but exhibited far lower acrolein selectivity values than the heteropoly acid catalysts, particularly the heteropoly acid catalyst that was heat treated at 220 °C for one hour. It can be seen in Table 2 of FIG. 4 that the pKa values of the heteropoly acid catalysts heat treated at high temperatures (400 °C to 550 °C) increased significantly from the pKa value of the heteropoly acid catalyst heat treated at 220 °C. This not only explains the decrease in activity and selectivity of these materials, but also indicates that a heteropoly acid catalyst should be maintained at lower temperatures such as, for example, a temperature range of approximately 150 °C to 250 °C.

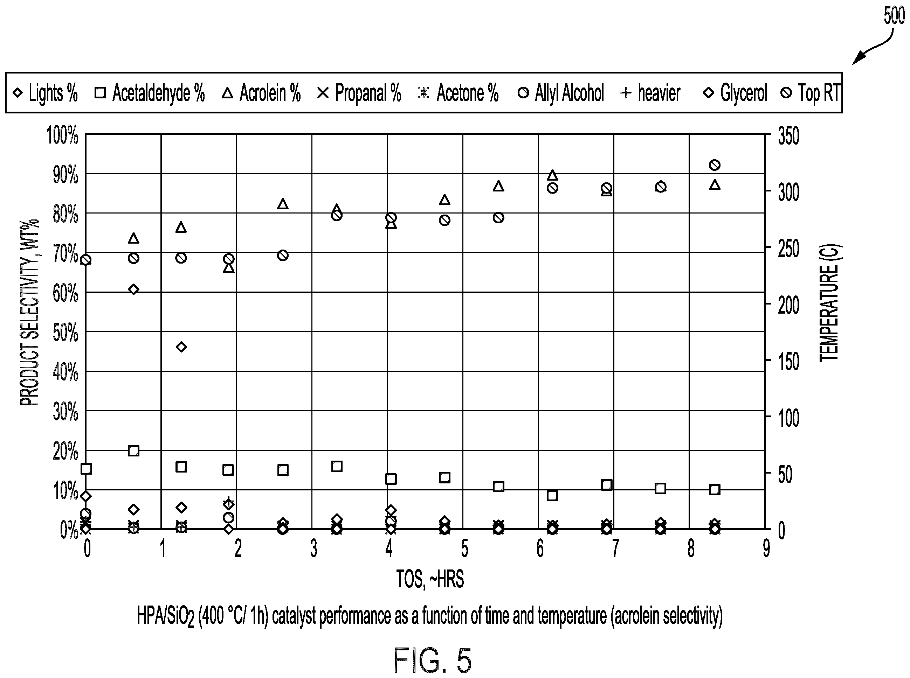

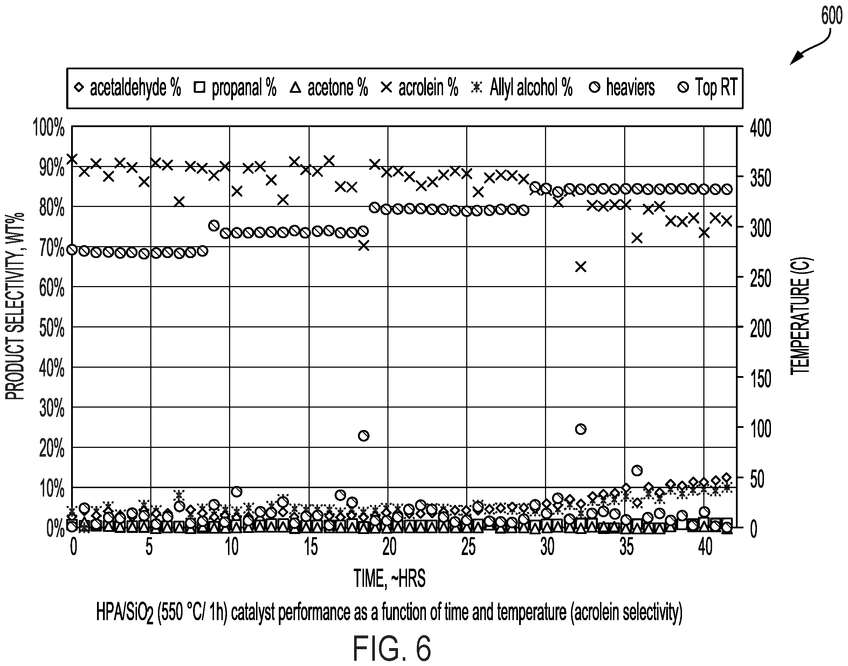

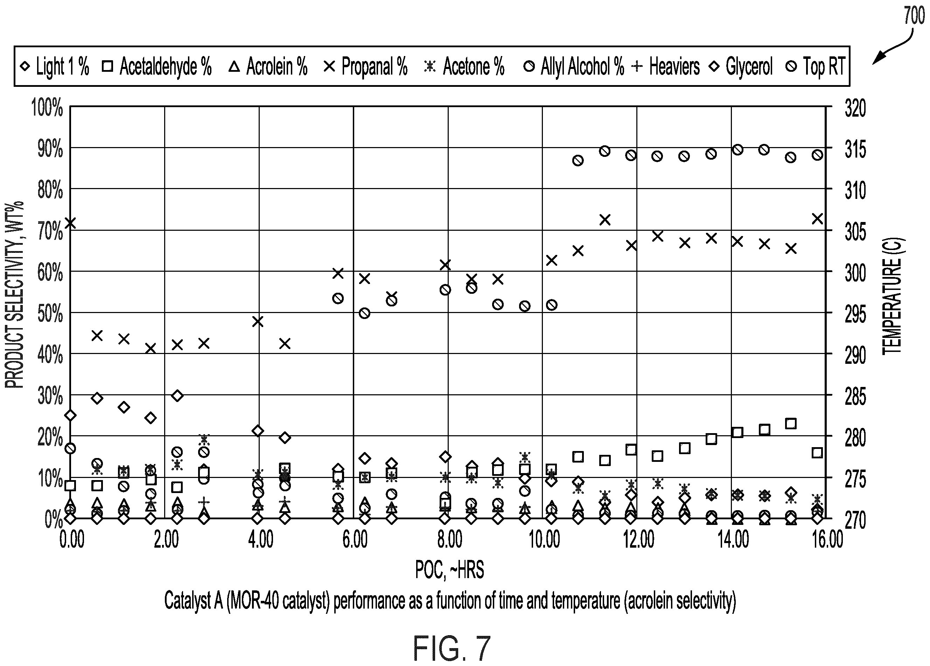

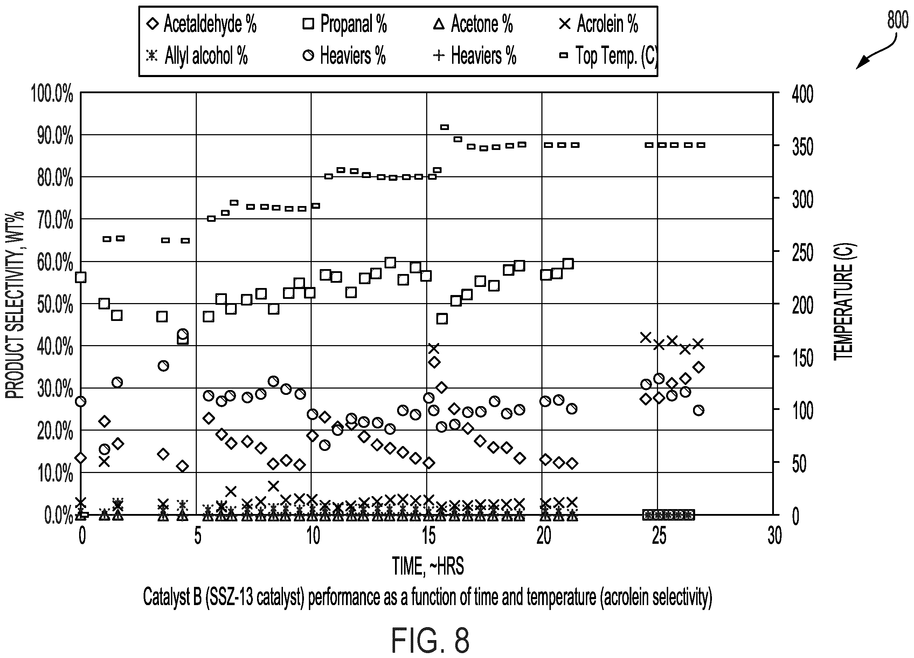

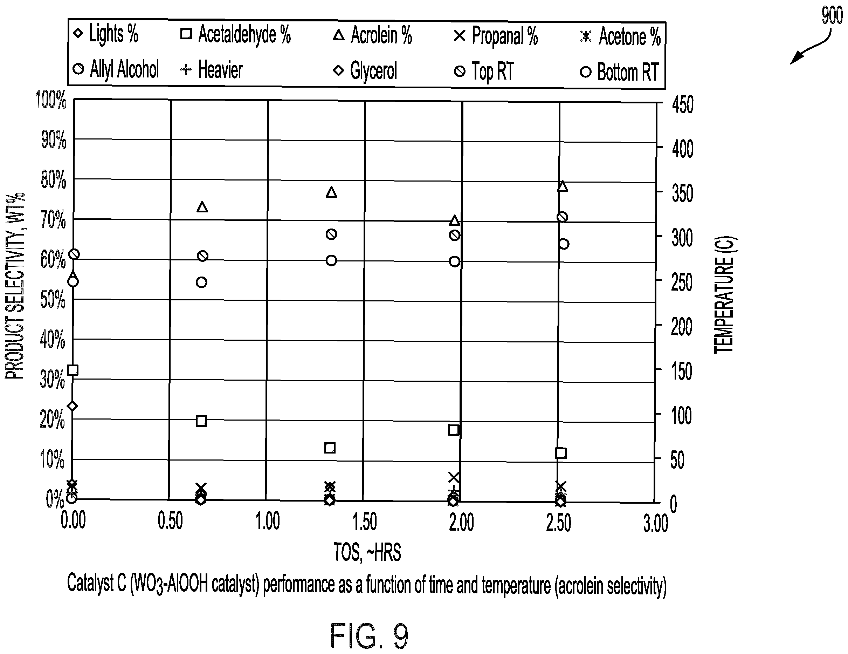

In addition to the numerical catalyst performance values presented in FIG. 4, the performance exhibited by each catalyst of Example 3 (except for the heteropoly acid catalyst heat treated at 220 °C) is graphically represented in FIGS. 5-9. Specifically, FIG. 5 is a graph 500 illustrating the acrolein selectivity of the heteropoly acid catalyst heat treated to 400 °C, FIG. 6 is a graph 600 illustrating the acrolein selectivity of the heteropoly acid catalyst heat treated to 550 °C, FIG. 7 is a graph 700 illustrating the acrolein selectivity of Catalyst A, FIG. 8 is a graph 800 illustrating the acrolein selectivity of Catalyst B and FIG. 9 is a graph 900 illustrating the acrolein selectivity of Catalyst C. Each of FIGS. 5-9 also indicate the selectivity of various byproducts produced by the acrolein production process using each of the corresponding catalysts.

Example 4 - Sulfonic Acid Catalyst Performance

In Example 4, the pilot plant 100 was again utilized to test the process for producing acrolein from glycerol as described above with respect to Example 1, but using a heterogenous catalyst in the form of a sulfonic acid catalyst comprising amorphous silica having organosulfonic acid groups chemically bonded thereto. The sulfonic acid catalyst in this example was prepared using ethylbenzenesulfonic acid. The acid dissociation constant (pKa) value of the ethylbenzenesulfonic acid catalyst was 3.4, and the particle sizes of the silica were between 500 $\mu$m to 800 $\mu$m. The ethylbenzenesulfonic acid catalyst was diluted 1:1 with silicon carbide (SiC) to facilitate effective flow distribution and heat transfer.

Once in the reactor 102, the ethylbenzenesulfonic acid catalyst was preheated to 220 °C and held at that temperature for one hour. The ethylbenzenesulfonic acid catalyst was then further heated to the target temperature for the dehydration reaction prior to the start thereof. Once the target reaction temperature was achieved, 20 weight percent of the aqueous glycerol was vaporized to form the vapor phase glycerol 114, which was then introduced to the reactor 102 in the same manner described with respect to the previous examples, and the reaction conditions were controlled to ensure that contact of the glycerol with the ethylbenzenesulfonic acid catalyst occurred in a vapor phase state. The weight hour space velocity (WHSV) was set at 3 h-1, the glycerol partial pressure was 3E-02 atmospheres, and the flow of glycerol was maintained at 3 gallons per hour. The reaction was performed at atmospheric pressure.

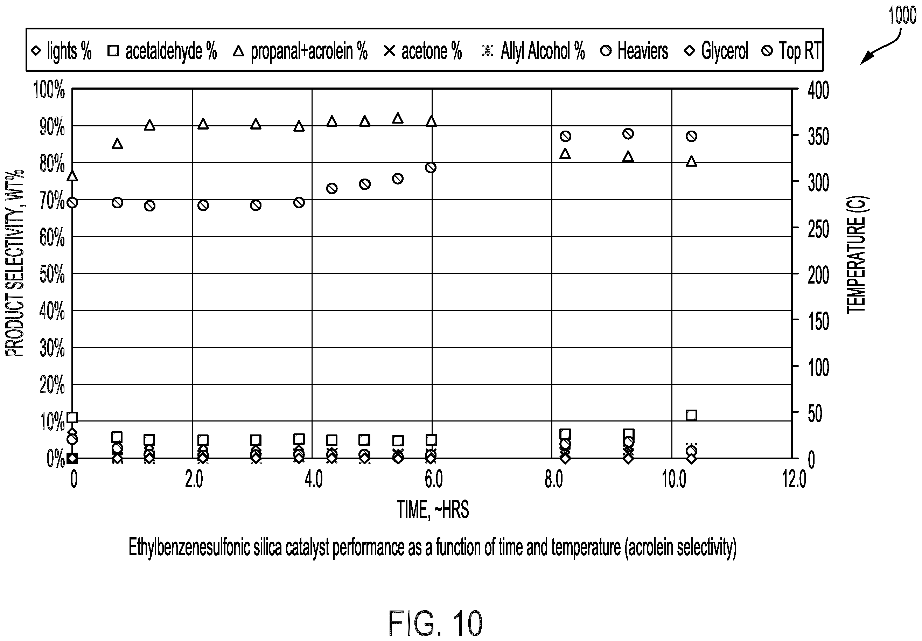

FIG. 10 is a graph 1000 illustrating the performance of silicon carbide-diluted ethylbenzenesulfonic acid catalyst relative to the production of acrolein from the glycerol 114. As shown, once the reactor 102 reached steady state, an acrolein selectivity of over 90% was achieved at a reaction temperature of between 280 °C to 315 °C. It may also be observed that the acrolein selectivity decreased almost 10% when the reaction temperature was increased to 350 °C. Analysis by gas chromatograph detected no glycerol in the reactor effluent 116. The primary by-products in the effluent 116 were determined to be acetaldehyde and acetone.

Example 5 - Effect of Acid Strength on Sulfonic Acid Catalyst Performance

In Example 5, the ethylbenzenesulfonic acid catalyst of Example 4 was tested for acrolein production performance against three other catalysts in accordance with the dehydration reaction procedure described in Example 4. In this example, the three other catalysts were once again the zeolite Mordenite Si/Al 40 (MOR 40) (Catalyst A), the zeolite Chabazite (SSZ-13) (Catalyst B), and the WO$_3$/AlOOH material (Catalyst C) described in Example 3. Acid dissociation constant (pKa) values for each catalyst were again measured by titration using a dispersion 0.15 grams of the catalyst in 40 grams of a sodium chloride/water solution (2.5 weight percent), and the resulting slurry was left for a minimum of 4 hours under agitation. Titration was subsequently performed using a solution of sodium hydroxide (0.1-0.01 N). The pKa value for each catalyst was determined at the half titration point in the titration curve, at which point the pKa value is equivalent to the pH value.

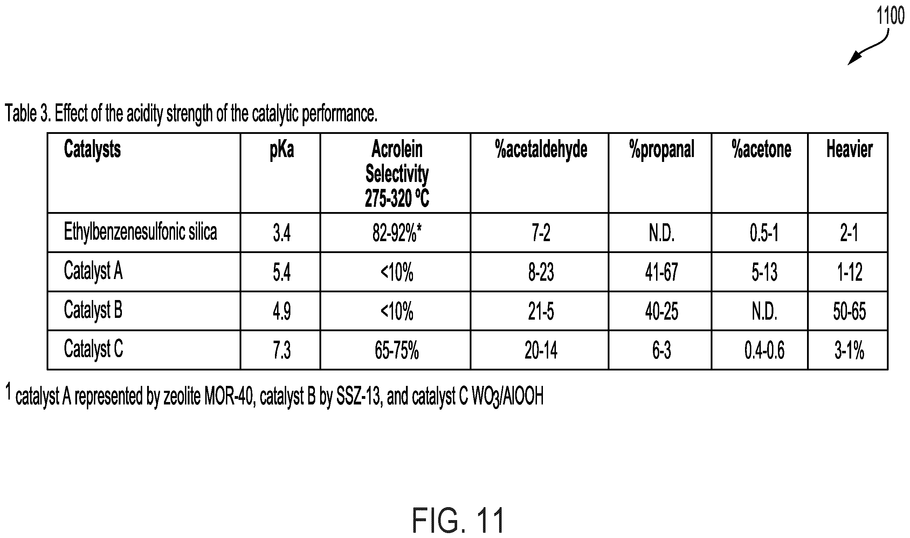

FIG. 11 includes a table 1100 of testing data and is labeled as Table 3. The average pKa value for each catalyst is reported in Table 3 in units of milliequivalents per gram (meq/g). The catalytic performance exhibited by each catalyst when used in a glycerol dehydration process at temperatures ranging from 275 °C to 320 °C is also reported in Table 3. As indicated, acrolein selectivity generally correlates strongly with the strength of the heteropoly acid catalyst. For example, it may be observed that the acrolein selectivity is high (between 82% to 92%) when the pKa value of the ethylbenzenesulfonic acid catalyst is relatively low (e.g., 3.4). In contrast, Catalyst A and Catalyst B, which once again had pKa values of 5.4 and 4.9 respectively, each had an acrolein selectively of less than 10%. While Catalyst C again had the highest pKa value of the tested materials, at 7.3, Catalyst C nonetheless exhibited better acrolein selectivity than Catalyst A and Catalyst B, but exhibited far lower acrolein selectivity values than the ethylbenzenesulfonic acid catalyst. The difference in the catalytic performance exhibited by the ethylbenzenesulfonic acid catalyst of this example relative to each of Catalyst A, Catalyst B, and Catalyst C, may be further understood by comparing the ethylbenzenesulfonic acid catalyst acrolein selectivity graph of FIG. 10 with the graphs of FIGS. 7-9, which respectively depict the acrolein selectivity performance of Catalyst A, Catalyst B, and Catalyst C.

Example 6 - Sulfonic Acid Catalyst Stability

In Example 6, a thermogravimetric analysis (TGA) was conducted to determine the thermal stability of the ethylbenzenesulfonic acid catalyst of Example 4 to determine when the sulfonic acid group substituent begins to decompose. For this example, the ethylbenzenesulfonic acid catalyst was initially heated for a period of time at 60 °C under vacuum to eliminate adsorbed water. The ethylbenzenesulfonic acid catalyst was then heated to 950 °C at a ramp rate of 5 °C/min while 20 milliliters per minute of nitrogen was passed over the ethylbenzenesulfonic acid catalyst.

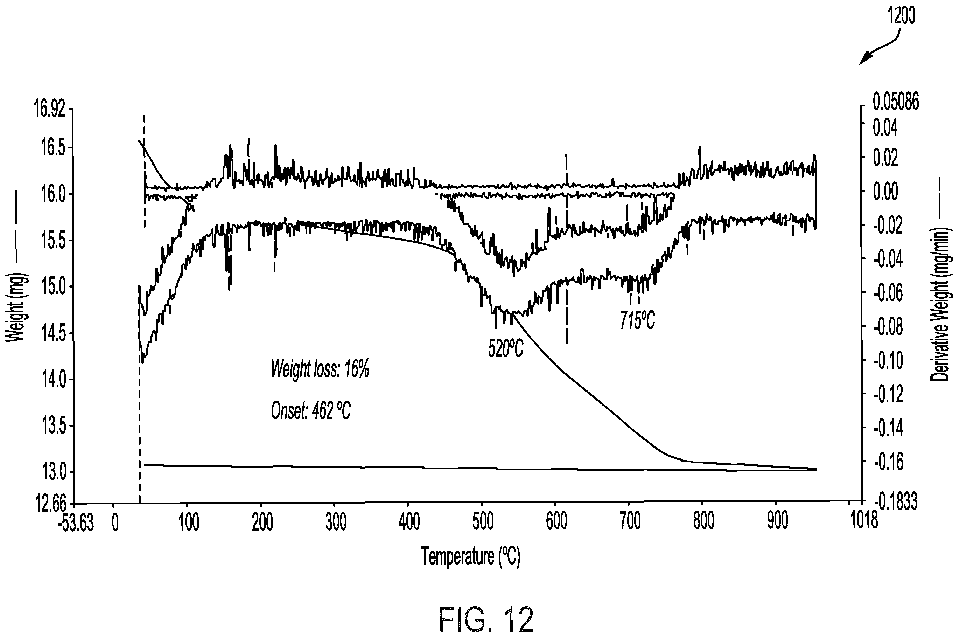

FIG. 12 is a TGA graph 1200 for the ethylbenzenesulfonic acid catalyst after heating according to the above procedure. As can be observed, the sulfonic acid groups were decomposed in two stages: a first group at a low temperature and a second group at a higher temperature. The temperatures of decomposition likely depend on the sulfonic species type and its interaction with the support and, therefore, may vary in other examples. In this example, the sulfonic acid groups of the ethylbenzenesulfonic acid catalyst began to decompose at about 462 °C. This is a higher temperature than the reaction temperature, which indicates that no sulfonic acid group decomposition occurred during the reaction.

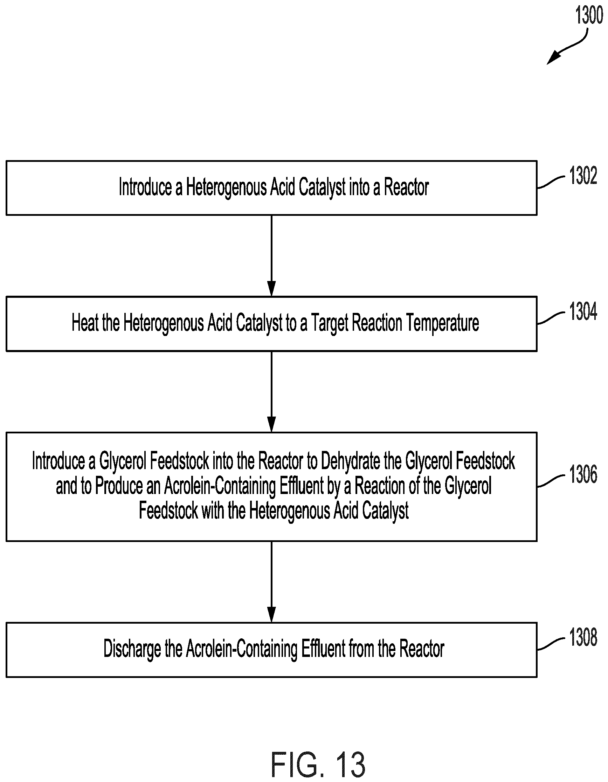

FIG. 13 is a flowchart 1300 of a method of producing acrolein from glycerol. As indicated in block 1302, the method can include introducing a heteropoly acid catalyst into a reactor. The reactor may include a fixed catalyst bed and a reactor-operated downflow. The reactor may be an adiabatic reactor or an isothermal reactor. In some examples, the heteropoly acid catalyst can be an amorphous silica having a heteropoly acid group. The heteropoly acid catalyst may have an acid dissociation constant (pKa) value of 3.7 or less.

As indicated in block 1304, the heteropoly acid catalyst may be preheated in the reactor for some period of time prior to further heating the heteropoly acid catalyst to the target reaction temperature. For example, the heteropoly acid catalyst may be preheated to a temperature between 150 °C to 250 °C. In one example, the heteropoly acid catalyst may be held at the predetermined temperature for one hour before the preheated heteropoly acid catalyst is further heated to the target reaction temperature.

As indicated in block 1306, the heteropoly acid catalyst can thereafter be further heated to a target reaction temperature. In one example, the target reaction temperature is between 220 °C to 300 °C.

As indicated in block 1308, glycerol can be introduced into the reactor to contact the heteropoly acid catalyst. The glycerol may be vapor phase glycerol generated, for example, by vaporizing a flow of aqueous glycerol in a vaporizer. In other examples, the aqueous glycerol may be introduced into the reactor in liquid form. Contact of the glycerol with the heteropoly acid catalyst causes a reaction within the reactor that dehydrates the glycerol and produces acrolein, which is contained in a gaseous or liquid effluent generated by the reaction.

As indicated in block 1310, the effluent can be discharged from the reactor. For example, the effluent can be discharged to one or more downstream cooling, distillation or separation processes. In some examples, these downstream processes may be used to convert the acrolein in the effluent into acrylic acid.

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure.

**Claims**

1. A method of producing acrolein from glycerol comprising:

   introducing into a reactor, a heteropoly acid catalyst comprising amorphous silica having a heteropoly acid group;
   preheating the heteropoly acid catalyst in the reactor to a temperature within a temperature range of between 150 °C to 250 °C and maintaining the temperature of the heteropoly acid catalyst within the temperature range for a predetermined period of time to produce a preheated heteropoly acid catalyst;
   further heating the preheated heteropoly acid catalyst to a target reaction temperature;
   introducing glycerol into the reactor and dehydrating the glycerol in the reactor by a reaction of the glycerol with the heteropoly acid catalyst that produces an acrolein-containing effluent; and
   discharging the acrolein-containing effluent from the reactor;
   optionally, wherein the target reaction temperature is between 220 °C to 300 °C.

2. The method of claim 1, wherein a pore volume (PV) of the heteropoly acid catalyst satisfies a formula:
   PV > 0.6-0.3 [loading/surface area of dried heterogenous acid catalyst], where PV is measured in milliliters per gram of catalyst, the loading is an amount of heterogenous acid present in the dried heterogenous acid catalyst and is measured in micromoles per gram, and the surface area of dried heterogenous acid catalyst is measured in square meters per gram of the heterogenous acid catalyst.

3. The method of claim 1, wherein the glycerol is vapor phase glycerol or aqueous glycerol.

4. The method of claim 1, wherein the heteropoly acid catalyst has an acid dissociation constant (pKa) value of 3.7 or less.

5. The method of claim 1, wherein the heteropoly acid catalyst is selected from the group consisting of tungsten-based heteropoly acids including tungstosilicic acid, tungstophosphoric acid, and phosphomolybdic acid, and salt-based heteropoly acids including 12-tungstosilicic acid and 18-tungstiphosphoric acid.

6. The method of claim 1, wherein the amorphous silica contains between zero to 0.05 weight percent of zirconium, between zero to 0.035 weight percent of sodium, between zero to 40 parts per million of iron, and between zero to 60 parts per million of molybdenum.

7. The method of claim 1, wherein the reaction occurs in a vapor phase state.

8. The method of claim 1, wherein a pressure in the reactor at a time of the reaction is at least 0.1 MPa below a dew point pressure, and at least 10 °C above a dew point temperature, of both the glycerol and the acrolein-containing effluent produced by the reaction.

9. The method of claim 1, wherein the glycerol contains water or is combined with water to regulate an endothermic reaction temperature profile inside the reactor.

10. A method of producing acrolein from glycerol comprising:

   introducing into a reactor, a sulfonic acid catalyst comprising amorphous silica having organosulfonic acid groups chemically bonded thereto and having an acid dissociation constant (pKa) value of 3.5 or less;
   heating the sulfonic acid catalyst to a target reaction temperature;
   introducing glycerol into the reactor and dehydrating the glycerol in the reactor by a reaction of the glycerol with the sulfonic acid catalyst that produces an acrolein-containing effluent; and
   discharging the acrolein-containing effluent from the reactor; optionally, wherein the glycerol is vapor phase glycerol or aqueous glycerol.

11. The method of claim 1, wherein the target reaction temperature is between 220 °C to 300 °C.

12. The method of claim 1, wherein the reaction occurs in a vapor phase state.

13. The method of claim 1, wherein a pressure in the reactor at a time of the reaction is at least 0.1 MPa below a dew point pressure, and at least 10 °C above a dew point temperature, of both the glycerol and the acrolein-containing effluent produced by the reaction.

14. The method of claim 1, wherein the glycerol contains water or is combined with water to regulate an endothermic reaction temperature profile inside the reactor.

15. A catalyst for producing acrolein from glycerol comprising:

   an amorphous silica having organosulfonic acid groups chemically bonded thereto; and
   wherein the catalyst has an acid dissociation constant (pKa) value of 3.5 or less; optionally, wherein the sulfonic acid catalyst is selected from the group consisting of methanesulfonic acid silica, ethanesulfonic acid silica, propanesulfonic acid silica, butanesulfonic acid silica, benzenesulfonic acid silica, ethylbenzenesulfonic acid silica, vinylbenzenesulfonic acid silica, propylbenzenesulfonic acid silica, and butylbenzene sulfonic acid silica; and/or wherein the amorphous silica of the sulfonic acid catalyst is functionalized with one or more organosulfonic acid groups having a formula $R^1SO_3H$, and the $R^1$ is chemically bonded to the amorphous silica and is selected from the group consisting of:

      a substituted alkyl group, a substituted alkenyl group, and a substituted alkynyl group, each having between 1 to 8 carbon atoms, and
      a substituted or unsubstituted aryl group; and/or wherein the amorphous silica comprises silica particles in the form of shaped objects in which the silica particles are composited together with or without the aid of a binder.

FIG. 1

HPA/SiO₂ (220 °C/ 1h) catalyst performance as a function of time and temperature (acrolein selectivity)

FIG. 2

Table 1. Effect of thermal treatment on the catalytic performance.

| Catalysts | Acrolein Selectivity 275-320 °C | %acetaldehyde | %propanal | %acetone | Heavier |
|---|---|---|---|---|---|
| HPA/SiO$_2$ (220 °C/1h) | 83-88% | 9-8 | 3-1 | 0.7-0.3 | 4-1 |
| HPA/SiO$_2$ (400 °C/1h) | 77-89% | 16-9 | 2-0.5 | 0.7-0.1 | 3-0.3 |
| HPA/SiO$_2$ (550 °C/1h) | 74-90% | 13-2 | 1-0.4 | 1.4-0.3 | 13-4 |

300

FIG. 3

Table 2. Effect of the acidity strength of the catalytic performance.

| | pKa | Acrolein Selectivity 275-320 ºC | %acetaldehyde | %propanal | %acetone | Heavier |
|---|---|---|---|---|---|---|
| HPA/SiO$_2$ (220 ºC/1h) | 3.6 | 83-88% | 9-8 | 3-1 | 0.7-0.3 | 4-1 |
| HPA/SiO$_2$ (400 ºC/1h) | 5.9 | 77-89% | 16-9 | 2-0.5 | 0.7-0.1 | 3-0.3 |
| HPA/SiO$_2$ (550 ºC/1h) | 6.1 | 74-90% | 13-2 | 1-0.4 | 1.4-0.3 | 13-4 |
| Catalyst A | 5.4 | <10% | 8-23 | 41-67 | 5-13 | 1-12 |
| Catalyst B | 4.9 | <10% | 21-5 | 40-25 | N.D. | 50-65 |
| Catalyst C | 7.3 | 65-75% | 20-14 | 6-3 | 0.4-0.6 | 3-1% |

400

FIG. 4

EP 4 700 008 A1

HPA/SiO₂ (400 °C/ 1h) catalyst performance as a function of time and temperature (acrolein selectivity)

FIG. 5

EP 4 700 008 A1

HPA/SiO$_2$ (550 °C/ 1h) catalyst performance as a function of time and temperature (acrolein selectivity)

FIG. 6

Legend: ◇ Light 1 %  □ Acetaldehyde %  △ Acrolein %  × Propanal %  ✳ Acetone %  ○ Allyl Alcohol %  + Heaviers  ◇ Glycerol  ⊘ Top RT

700

Catalyst A (MOR-40 catalyst) performance as a function of time and temperature (acrolein selectivity)

FIG. 7

Catalyst B (SSZ-13 catalyst) performance as a function of time and temperature (acrolein selectivity)

FIG. 8

EP 4 700 008 A1

FIG. 9

Catalyst C (WO₃-AlOOH catalyst) performance as a function of time and temperature (acrolein selectivity)

Ethylbenzenesulfonic silica catalyst performance as a function of time and temperature (acrolein selectivity)

FIG. 10

Table 3. Effect of the acidity strength of the catalytic performance.

| Catalysts | pKa | Acrolein Selectivity 275-320 ℃ | %acetaldehyde | %propanal | %acetone | Heavier |
|---|---|---|---|---|---|---|
| Ethylbenzenesulfonic silica | 3.4 | 82-92%* | 7-2 | N.D. | 0.5-1 | 2-1 |
| Catalyst A | 5.4 | <10% | 8-23 | 41-67 | 5-13 | 1-12 |
| Catalyst B | 4.9 | <10% | 21-5 | 40-25 | N.D. | 50-65 |
| Catalyst C | 7.3 | 65-75% | 20-14 | 6-3 | 0.4-0.6 | 3-1% |

[1] catalyst A represented by zeolite MOR-40, catalyst B by SSZ-13, and catalyst C $WO_3/AlOOH$

FIG. 11

FIG. 12

1300

Introduce a Heterogenous Acid Catalyst into a Reactor — 1302

Heat the Heterogenous Acid Catalyst to a Target Reaction Temperature — 1304

Introduce a Glycerol Feedstock into the Reactor to Dehydrate the Glycerol Feedstock and to Produce an Acrolein-Containing Effluent by a Reaction of the Glycerol Feedstock with the Heterogenous Acid Catalyst — 1306

Discharge the Acrolein-Containing Effluent from the Reactor — 1308

FIG. 13

## EUROPEAN SEARCH REPORT

**Application Number**

EP 24 30 6368

### DOCUMENTS CONSIDERED TO BE RELEVANT

| Category | Citation of document with indication, where appropriate, of relevant passages | Relevant to claim |
|---|---|---|
| X | CN 102 695 694 A (ADISSEO FRANCE SAS; CENTRE NAT RECH SCIENT ET AL.) 26 September 2012 (2012-09-26) | 1-9, 11-14 |
| A | * paragraph [0033] - paragraph [0034]; claim 1 * | 10 |
| | ----- | |
| X | WO 2022/103394 A1 (BADGER LICENSING LLC [US]) 19 May 2022 (2022-05-19) * Examples; claim 1 * | 15 |
| | ----- | |

**CLASSIFICATION OF THE APPLICATION (IPC)**

INV.
C07C45/52
C07C47/22
B01J21/08
B01J27/02

**TECHNICAL FIELDS SEARCHED (IPC)**

C07C
B01J

The present search report has been drawn up for all claims

| Place of search | Date of completion of the search | Examiner |
|---|---|---|
| The Hague | 2 February 2025 | Fritz, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 6368

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2025

| Patent document cited in search report | | Publication date | Patent family member(s) | | Publication date |
|---|---|---|---|---|---|
| CN 102695694 | A | 26-09-2012 | BR | 112012014883 A2 | 27-03-2018 |
| | | | CN | 102695694 A | 26-09-2012 |
| | | | EP | 2516375 A1 | 31-10-2012 |
| | | | FR | 2954312 A1 | 24-06-2011 |
| | | | JP | 2013515044 A | 02-05-2013 |
| | | | KR | 20120097540 A | 04-09-2012 |
| | | | TW | 201130791 A | 16-09-2011 |
| | | | US | 2012330049 A1 | 27-12-2012 |
| | | | WO | 2011083254 A1 | 14-07-2011 |
| WO 2022103394 | A1 | 19-05-2022 | CN | 116457092 A | 18-07-2023 |
| | | | EP | 4243980 A1 | 20-09-2023 |
| | | | JP | 2023550321 A | 01-12-2023 |
| | | | KR | 20230101904 A | 06-07-2023 |
| | | | TW | 202237264 A | 01-10-2022 |
| | | | US | 2024001352 A1 | 04-01-2024 |
| | | | WO | 2022103394 A1 | 19-05-2022 |

EPO FORM P0459

For more details about this annex : see Official Journal of the European Patent Office, No. 12/82